# EUROPÄISCHE PATENTSCHRIFT

(11) **EP 1 021 125 B1**
(45) Veröffentlichungstag und Bekanntmachung des Hinweises auf die Patenterteilung: **31.03.2004**
(21) Anmeldenummer: 98951509.3
(22) Anmeldetag: 12.10.1998
(51) Int. Cl.: A61B 1/267

(54) **LARYNGOSKOP**
LARYNGOSCOPE
LARYNGOSCOPE

(30) Priorität: 11.10.1997 GB 9721533; 17.06.1998 GB 9812973
(43) Veröffentlichungstag der Anmeldung: 26.07.2000
(73) Patentinhaber: Karl Storz GmbH & Co. KG, 78532 Tuttlingen (DE)
(72) Erfinder: HENDERSON, John, J., Milngavie, Glasgow G62 7RE (GB)
(74) Vertreter: Heuckeroth, Volker, Dr.
(86) Internationale Anmeldenummer: PCT/EP1998/006438
(87) Internationale Veröffentlichungsnummer: WO 1999/018841

(56) Entgegenhaltungen:
- EP-A- 0 184 588
- DE-A- 3 217 476
- US-A- 3 856 001
- US-A- 4 527 553
- US-A- 5 263 472

## Beschreibung

Die Erfindung betrifft ein Laryngoskop, insbesondere zum Einführen eines Tubus in die Luftröhre, mit einem sich im wesentlichen gerade erstreckenden Laryngoskopspatel, und mit einem Handgriff, wobei der Laryngoskopspatel zumindest teilweise als rohrförmiger Hohlkörper zum Durchführen eines Tubus ausgebildet ist, der eine seitliche, sich von einem proximalen Ende zu einem distalen Ende des rohrförmigen Hohlkörpers erstreckende Längsöffnung aufweist, wobei sich an das distale Ende des rohrförmigen Hohlkörpers eine Spitze anschließt.

Ein derartiges Laryngoskop ist aus dem Dokument US-A-4 527 553 bekannt.

Ein Laryngoskop ist ein medizinisches Instrument, das der Sichtbeobachtung des Kehlkopfes (Larynx) dient. In der Anästhesie wird ein solches Laryngoskop gleichzeitig dazu verwendet, einen Tubus, beispielsweise einen Narkosetubus, mit Hilfe des Laryngoskops unter Sichtbeobachtung in die Luftröhre eines Patienten einzuführen, um den Patienten während der Narkose künstlich beatmen zu können. Mit einem Laryngoskop kann das Einführen eines Tubus in die Luftröhre wesentlich vereinfacht werden.

Das erste Laryngoskop wurde 1895 beschrieben, wobei ein Laryngoskop zum Intubieren eines Patienten unter Sichtkontrolle erstmals 1912 eingesetzt wurde. Seit dieser Zeit ist der Einsatz von Laryngoskopen zur Luftröhrenintubation unter Sichtkontrolle ein wesentlicher Teil der Praxis in der Anästhesie und in der Intensivmedizin geworden.

Bis 1943 wurden Laryngoskope mit geraden Laryngoskopspateln verwendet. 1943 beschrieb Mackintosh erstmals ein Laryngoskop mit einem gekrümmten Laryngoskopspatel, das mit einer neuen Einsetztechnik in der Laryngoskopie verwendet wurde. Seitdem hat sich die Verwendung des gekrümmten Laryngoskops als Standardverfahren durchgesetzt. Dies deswegen, weil bei einem durchschnittlichen Patienten das Verfahren nach Mackintosh leichter durchzuführen ist als ein Verfahren, bei dem ein Laryngoskop mit geradem Laryngoskopspatel verwendet wird. Ein weiterer Grund liegt darin, daß viele Anästhesisten mit einem geraden Laryngoskop nicht das optimale Verfahren fanden, weil sie das Laryngoskop mittig in den Mund über den Zungenrücken eingeführt haben. Dieses frühere Verfahren ist bei den meisten schwierigen Patienten wenig erfolgreich.

Das Verfahren nach Mackintosh hat sich dagegen als sehr erfolgreich erwiesen. Unglücklicherweise ermöglicht es jedoch nicht die Sichtbeobachtung des Kehlkopfes bei 2 bis 5 % der Patienten. Bei diesen Patienten kann die Luftröhrenintubation ohne Sichtkontrolle in einem "Blind"-Verfahren erreicht werden. Blindverfahren bergen jedoch mehrere Risiken in sich, einschließlich eines erheblichen Traumas von beteiligtem Gewebe.

Probleme bei der Luftröhrenintubation sind ein Hauptgrund von Todesfällen unter Anästhesie. Es wurde geschätzt, daß in der westlichen Welt jährlich 600 Todesfälle als Folge von Problemen bei der Luftröhrenintubation auftreten. Zusätzlich können weitere erhebliche Komplikationen, so wie Schädigung des Hirns, der Luftröhre, der Speiseröhre und anderer Gewebe zu Todesfällen führen.

Es gibt eine Anzahl von Möglichkeiten, mit den Schwierigkeiten bei der Intubation fertigzuwerden, jedoch weisen alle Blindverfahren signifikante Fehlerraten auf. Das ideale Verfahren sollte relativ einfach sein, eine einfache Ausrüstung verwenden und eine große Erfolgs- und eine niedrige Fehlerrate besitzen.

Ein optimales Verfahren (paraglossale gerade Laryngoskopie) der Luftröhrenintubation für diese schwierigen Patienten ist 1997 von dem Erfinder der vorliegenden Anmeldung beschrieben worden. Dieses Verfahren weist noch eine hohe Erfolgsrate auf, wenn das Verfahren nach Mackintosh fehlschlägt. Unglücklicherweise ist derzeit kein Laryngoskop verfügbar, das für dieses Verfahren ohne Nachteile einsetzbar wäre.

Bei der schwierigen Luftröhrenintubation bestehen zwei Probleme. Das erste Problem besteht darin, den Kehlkopf beobachten zu können, das zweite darin, einen Tubus durch den Kehlkopf und in die Luftröhre zu führen.

Das am häufigsten verwendete gerade Laryngoskop ist das Laryngoskop nach Miller. Wenn dieses Laryngoskop bei dem paraglossalen Verfahren verwendet wird, ist es gewöhnlich erfolgreich, wenn das Verfahren nach Mackintosh fehlschlägt. Die Manipulation des einzuführenden Tubus erfordert es jedoch, einen zusätzlichen Raum entlang des Tubus zu schaffen. Dies erfordert einen größeren Hebedruck. Eine solche größere Hebekraft kann nicht immer erreicht werden, und kann außerdem traumatisch wirken. Zusätzlich beeinträchtigt die Ausgestaltung des Laryngoskops selbst die Handhabung des einzuführenden Tubus.

Weitere Laryngoskope sind bekannt (beispielsweise nach Kleinsasser, Holinger, Benjamin), die eine gute Sichtbeobachtung des Kehlkopfes bei schwierigen Patienten ermöglichen, jedoch ist das Einführen des Tubus bei diesen aufwendig und erfordert mehrere Schritte, und es beansprucht viel Zeit, was bei schwierigen Patienten kritisch sein kann.

Ein weiteres bekanntes Laryngoskop, das PCV-Laryngoskop, ermöglicht ein leichtes Einführen des Tubus. Unglücklicherweise ist jedoch die Ausgestaltung dieses Laryngoskopes nicht optimal. Dieses Laryngoskop weist eine Krümmung auf, die zwar das Einsetzen des Laryngoskops begünstigt, jedoch nur eine schlechte Sichtbeobachtung des Kehlkopfes bei den meisten schwierigen Patienten ermöglicht. Die Ausgestaltung der Spitze beeinträchtigt außerdem eine optimale Kontrolle des Kehldeckels (Epiglottis). Der Lichtträger ragt in die Unterseite des Laryngoskops vor, an der der größte Gewebedruck von dem Laryngoskopspatel ausgeübt wird, und erhöht somit das Risiko eines Gewebetraumas. Das PVC-Laryngoskop ist außerdem kostenintensiv.

Wie bereits eingangs erwähnt, ist das am meisten verwendete Laryngoskop nach Mackintosh in seiner Längsachse gekrümmt. Es nimmt jedoch die Erkenntnis zu, daß eine derartige Ausgestaltung mit einer höheren Fehlerrate bei der Luftröhrenintubation als bei Laryngoskopen mit geraden Laryngoskopspateln verbunden ist.

Das aus dem eingangs genannten Dokument US-A-4 527 553 bekannte Laryngoskop weist einen Laryngoskopspatel auf, an den sich an seinem distalen Ende eine Spitze anschließt, die in Richtung quer zur Längsrichtung des Laryngoskopspatels gewölbt ist, die an ihrem distalen Ende abgerundet ist und außerdem spitz zuläuft.

Der Erfindung liegt die Aufgabe zugrunde, ein Laryngoskop der eingangs genannten Art dahingehend weiterzubilden, daß das Laryngoskop eine verbesserte Sichtkontrolle des Kehlkopfes und gleichzeitig ein verbessertes Durchführen eines Tubus durch den Kehlkopf in die Luftröhre ermöglicht.

Erfindungsgemäß wird diese Aufgabe hinsichtlich des eingangs genannten Laryngoskops dadurch gelöst, daß die Spitze in Richtung quer zur Längsrichtung des Laryngoskopspatels eben und flach ist und ihr distales Ende in Richtung quer zur Längsrichtung im wesentlichen gerade ausgeführt ist.

Gegenüber den oben beschriebenen bekannten Laryngoskopen hat die erfindungsgemäße rohrförmige Ausgestaltung des Laryngoskopspatels den Vorteil, daß das Einsetzen eines Tubus mit Hilfe des Laryngoskops in die Luftröhre wesentlich vereinfacht ist. Der Tubus kann in einem Grundverfahren entweder direkt durch das Lumen des Laryngoskopspatels oder über eine Einführhilfe ebenfalls durch das Lumen des Laryngoskopspatels unter Sichtkontrolle geführt werden. Bei dieser durch die rohrförmige Ausgestaltung des Laryngoskopspatels ermöglichten Art des Einführens des Tubus ist es im Unterschied zu den bekannten Laryngoskopen nicht erforderlich, seitlich des Laryngoskops einen Raum freizuschaffen, so daß eine geringere Hebekraft ausgeübt werden muß. Folglich kann das Einführen des Tubus weniger traumatisch erfolgen und kann auch bei schwierigen Patienten erfolgreich durchgeführt werden. Der Laryngoskopspatel ist jedoch nicht als geschlossen rohrförmiger Hohlkörper ausgebildet, sondern weist in Längsrichtung eine Längsöffnung auf. Der Laryngoskopspatel gemäß der vorliegenden Erfindung kann demnach als geschlitztes Rohr beschrieben werden. Die seitliche Längsöffnung führt zu weiteren wesentlichen Vorteilen der Erfindung. Zum einen ermöglicht die seitliche Längsöffnung eine verbesserte Sichtbeobachtung des Kehlkopfbereiches beim Einführen des Tubus. Die Längsöffnung vergrößert das Blickfeld, so daß eine binokulare Beobachtung ermöglicht wird. Zum anderen erleichtert die seitliche Längsöffnung das Entfernen des Laryngoskopes, nachdem der Tubus eingesetzt wurde, da der Laryngoskopspatel seitlich von dem Tubus abgeschält werden kann. Weiterhin ermöglicht die seitliche Längsöffnung auch ein Einführen des Tubus in den Laryngoskopspatel von der Seite her, also nicht nur vom proximalen Ende des Laryngoskopspatels aus, wenn die Längsöffnung in Umfangsrichtung entsprechend dimensioniert ist. In Verbindung mit der geraden Ausführung des Laryngoskopspatels wird eine optimale Beobachtung des Kehlkopfes auch bei schwierigen Patienten erreicht.

Erfindungsgemäß verjüngt sich der Laryngoskopspatel am distalen Ende zu einer quer zur Längsrichtung des Laryngoskopspatels ebenen und flachen Spitze.

Am distalen Ende ist der Laryngoskopspatel demnach nicht rohrförmig sondern flach ausgebildet. Dies ist einerseits von Vorteil, um eine optimale Kontrolle des Kehldeckels (Epiglottis) zu erreichen. Andererseits wird das Blickfeld bei der Sichtbeobachtung, insbesondere bei der Sichtbeobachtung des Kehlkopfes, weiter vergrößert und verbessert die Sichtkontrolle des Einführens des Tubus.

In einer bevorzugten Ausgestaltung ist der Laryngoskopspatel im Querschnitt etwa rund.

Hierbei ist von Vorteil, daß der in die Luftröhre einzuführende Tubus, der üblicherweise im Querschnitt rund ist, in dem Laryngoskopspatel leichter geführt werden kann.

In einer weiteren bevorzugten Ausgestaltung verjüngt sich der Laryngoskopspatel in einem proximalen Bereich im Querschnitt.

Da der Laryngoskopspatel in den Mund des Patienten eingesetzt wird, wobei der proximale Bereich zwischen den Zähnen des Patienten zu liegen kommt, wird hierdurch der Vorteil erzielt, daß das Risiko einer Zahnbeschädigung vermindert wird. Durch die sich verjüngende Ausgestaltung des proximalen Bereichs des Laryngoskopspatels wird das Auftreten einer Berührung des Laryngoskopspatels mit den Zähnen beim Heben oder Senken des Laryngoskopspatels vermindert.

In einer weiteren bevorzugten Ausgestaltung weitet sich die Längsöffnung zum proximalen Ende hin auf.

Hierbei ist von Vorteil, daß der Tubus leichter in das Lumen des Laryngoskopspatels eingeführt werden kann. Weiterhin erleichtert die Aufweitung der Längsöffnung das Abschälen des Laryngoskops von dem Tubus, nachdem der Tubus in die Luftröhre eingesetzt worden ist.

In einer weiteren bevorzugten Ausgestaltung weist die Längsöffnung im Querschnitt einen Öffnungswinkel im Bereich von etwa 180° bis etwa 90° auf.

In diesem Winkelbereich des Öffnungswinkels der Längsöffnung im Querschnitt gesehen kann der einzuführende Tubus einerseits sicher in dem Laryngoskopspatel gehalten und geführt werden, andererseits ermöglicht die Längsöffnung eine Vergrößerung des Blickfeldes und erleichtert das Abnehmen des Laryngoskopspatels von dem Tubus nach dem Einsetzen des Tubus in die Luftröhre.

Weiterhin ist es bevorzugt, wenn die Spitze geringfügig gekrümmt oder gerade ist.

Auch diese Maßnahmen führen zu einer verbesserten Kontrolle des Kehldeckels. Der Laryngoskopspatel geht demnach im distalen Bereich von einem rohrförmigen Querschnitt zu einem plattenförmigen Querschnitt über.

In einer weiteren bevorzugten Ausgestaltung erstreckt sich die Spitze etwa über die gesamte Breite des Laryngoskopspatels.

Hierbei ist von Vorteil, daß die Spitze flächig ausgebildet ist, so daß die Gefahr eines Gewebetraumas im Kehlkopfbereich weiter vermindert wird.

In einer weiteren bevorzugten Ausgestaltung weist die Spitze eine wulstartige Verdickung auf.

Hierbei ist von Vorteil, daß die Gefahr einer Verletzung von weichem Gewebe weiter vermindert wird.

In einer weiteren bevorzugten Ausgestaltung ist entlang des Laryngoskopspatels ein Lichtleiter geführt, dessen lichtaustrittsseitiges Ende im Lumen des Laryngoskopspatels mündet.

Bei dieser Ausgestaltung ist demnach der Lichtleiter, beispielsweise ein in einem Röhrchen aufgenommenes Faserbündel, vom proximalen Ende des Laryngoskopspatels zu dessen distalem Ende des Laryngoskopspatels geführt und mündet das lichtaustrittsseitige Ende des Lichtleiters am distalen Ende des Laryngoskopspatels im Lumen des Laryngoskopspatels, wodurch die Beleuchtung des Beobachtungsgebietes im Kehlkopfbereich weiter vorteilhaft verbessert wird, weil es nicht zu Behinderungen der Lichtausbreitung durch an dem Laryngoskopspatel anliegendes Gewebe, insbesondere weiches Gewebe, kommt. Das lichtaustrittsseitige Ende des Lichtleiters ist so gerichtet, daß es eine optimale Beleuchtung des Kehlkopfes bewirkt.

In einer weiteren bevorzugten Ausgestaltung weist das proximale Ende des Laryngoskopspatels einen Befestigungsabschnitt zum Verrasten des Handgriffs an dem Laryngoskopspatel auf.

Hierdurch wird der Vorteil erzielt, daß der Laryngoskopspatel als Einwegteil ausgebildet werden kann, wobei dann der Handgriff, der die Lichtquelle enthält, wiederverwendet werden kann. Es ist aber auch möglich, den Laryngoskopspatel nicht abnehmbar mit dem Handgriff befestigt auszugestalten. Es können Standardhandgriffe mit einer Faseroptik verwendet werden. Bei der Verwendung eines Standardhandgriffes kann das erfindungsgemäße Laryngoskop kostengünstig hergestellt werden.

In einer weiteren bevorzugten Ausgestaltung ist das Lumen des Laryngoskopspatels an den Durchmesser des einzuführenden Tubus angepaßt.

Hierbei ist von Vorteil, daß das erfindungsgemäße Laryngoskop jeweils an die spezielle Größe von Tuben angepaßt ist. Es können demnach verschiedene Laryngoskope mit unterschiedlichen Bemaßungen des Laryngoskopspatelquerschnitts bereitgehalten werden, um jeweils das optimale Laryngoskop für den jeweiligen Einsatzfall zur Verfügung zu haben.

In einer weiteren bevorzugten Ausgestaltung ist am Laryngoskopspatel ein Insufflationskanal angeordnet, der am distalen Ende des Laryngoskopspatels mündet.

Dieser Insufflationskanal kann vorteilhafterweise dazu verwendet werden, Sauerstoff zu insufflieren, bspw. einen intermittierenden Sauerstoffstrahl unter hohem Druck abzugeben, so daß eine Notfallbeatmung durchgeführt werden kann, wenn eine Verzögerung beim Einführen des Tubus eintritt. Der Insufflationskanal kann ebenso dazu verwendet werden, Lokalanästhetika auf den Kehlkopf und andere Organe im Luftweg des Patienten abzugeben.

Weitere Vorteile ergeben sich aus der nachfolgenden Beschreibung und der beigefügten Zeichnung.

Es versteht sich, daß die vorstehend genannten und die nachstehend noch zu erläuternden Merkmale nicht nur in ihrer jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden hiernach mit Bezug auf die Figuren näher beschrieben. Es zeigen:
- Fig. 1: ein Laryngoskop gemäß einem ersten Ausführungsbeispiel in einer Seitenansicht der rechten, geschlossenen Seite des Laryngoskopspatels;
- Fig. 2: einen Schnitt durch das Laryngoskop in Fig. 1 entlang der Linie II-II bei abgenommenem Handgriff;
- Fig. 3: einen Schnitt durch das Laryngoskop in Fig. 1 entlang der Linie III-III;
- Fig. 4: eine Seitenansicht auf die linke, offene Seite des Laryngoskops in Fig. 1;
- Fig. 5: eine Ansicht von unten auf das Laryngoskop in Fig. 1;
- Fig. 6: ein Laryngoskop gemäß einem weiteren Ausführungsbeispiel in einer Seitenansicht auf die rechte Seite des Laryngoskops ohne Handgriff;
- Fig. 7: eine Seitenansicht auf die linke Seite des Laryngoskops in Fig. 6;
- Fig. 8: eine Ansicht von unten auf das Laryngoskop in Fig. 6;
- Fig. 9: einen Schnitt entlang der Linie IX-IX in Fig. 6;
- Fig. 10: einen Schnitt entlang der Linie X-X in Fig. 6;
- Fig. 11: eine den Figuren 4 und 7 entsprechende Seitenansicht eines Laryngoskops gemäß einem weiteren Ausführungsbeispiel;
- Fig. 12: eine den Figuren 4, 7 und 11 entsprechende Seitenansicht eines Laryngoskops gemäß einem noch weiteren Ausführungsbeispiel;
- Fig. 13: eine Seitenansicht entsprechend Figuren 11 und 12 eines Laryngoskops gemäß einem noch weiteren Ausführungsbeispiel; und
- Fig. 14: einen Schnitt durch das Laryngoskop in Fig. 13 entlang der Linie XIV-XIV.

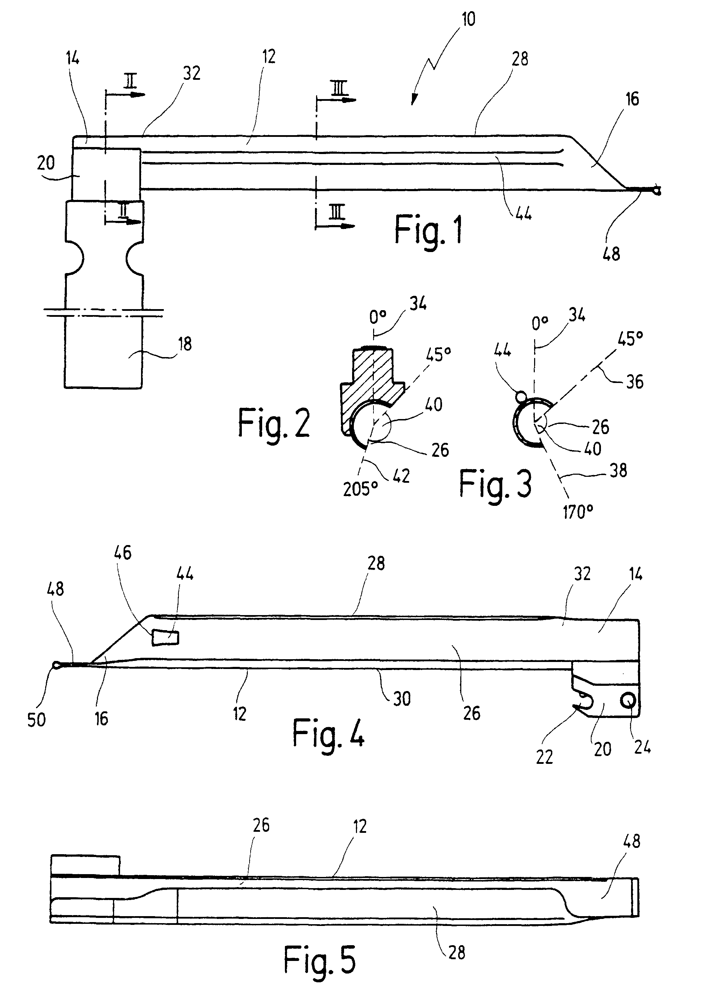

In Figuren 1 bis 5 ist ein mit dem allgemeinen Bezugszeichen 10 versehenes Laryngoskop dargestellt. Das Laryngoskop 10 wird als Inspektionsinstrument in den Mund- und Rachenraum eines Patienten zur Kehlkopfinspektion eingeführt. Für Anästhesiezwecke dient das Laryngoskop 10 weiterhin dazu, unter Sichtkontrolle einen Tubus mittels des Laryngoskops 10 in die Luftröhre eines Patienten einzuführen, um den Patienten während der Narkose über den Tubus künstlich zu beatmen.

Das Laryngoskop 10 weist einen Laryngoskopspatel 12 auf. Der Laryngoskopspatel 12 weist ein proximales Ende 14 und ein distales Ende 16 auf.

Am proximalen Ende 14 ist der Laryngoskopspatel 12 mit einem Handgriff 18 verbunden, der von dem Laryngoskopspatel 12 etwa orthogonal absteht.

Der Handgriff 18 ist von dem Laryngoskopspatel 12 abnehmbar. Am proximalen Ende 14 des Laryngoskopspatels 12 ist dafür ein Befestigungsabschnitt 20 angeordnet, der eine Verrastung des Handgriffs 18 mit dem Laryngoskopspatel 12 ermöglicht. Der Befestigungsabschnitt 20 weist eine oblong ausgebildete Ausnehmung 22 auf, in die ein Querstift des Handgriffs 18 (nicht dargestellt) einsetzbar ist. Am proximalen Ende des Befestigungsabschnitts 20 sitzt eine Raste 24 in Form einer mittels einer Feder nach außen gespannten Kugel, die in eine entsprechende kalottenförmige Ausnehmung im Handgriff 18 einrastet. Zum Montieren des Handgriffs 18 an den Laryngoskopspatel 12 wird zunächst der Stift in die Ausnehmung 22 eingesetzt, und anschließend wird der Handgriff nach hinten verschwenkt, so daß die Raste 24 in den Handgriff 18 einrastet.

Es ist anstelle eines abnehmbaren Handgriffs 18 aber auch möglich, einen Handgriff nicht abnehmbar mit dem Laryngoskopspatel 12 verbunden auszugestalten.

Der Laryngoskopspatel erstreckt sich im wesentlichen gerade, wie ein Vergleich der Darstellungen in Fig. 1 und Fig. 5 zeigt. Der Laryngoskopspatel 12 ist weiterhin als rohrförmiger Hohlkörper ausgebildet. Der Laryngoskopspatel 12 weist, wie aus Fig. 4 ersichtlich ist, eine seitliche, sich von dem proximalen Ende 14 zu dem distalen Ende 16 erstreckende Längsöffnung 26 auf. Die Längsöffnung 26 ist in Umfangsrichtung des Laryngoskopspatels 12 begrenzt. Eine Unterseite 28 (vgl. Fig. 5), die auch als oropharynegale Seite bezeichnet wird, ist geschlossen, ebenso eine Oberseite 30 (epiglottale Seite) des Laryngoskopspatels 12. Die Längsöffnung 26 befindet sich somit auf einer Seite, und zwar auf der linken Seite des Laryngoskopspatels 12. In Fig. 1 blickt man demnach auf die geschlossene rechte Seite des Laryngoskopspatels 12, während man in Fig. 4 in die offene Seite, d.h. in die Längsöffnung 26 hinein blickt.

Der Laryngoskopspatel 12 ist, wie aus Figuren 2 und 3 hervorgeht, im Querschnitt rund ausgebildet.

In einem proximalen Bereich 32 des Laryngoskopspatels 12 verjüngt sich dieser geringfügig im Querschnitt.

Die Längsöffnung 26 weitet sich zum proximalen Ende 14 hin auf. Die Aufweitung der Längsöffnung 26 erfolgt im proximalen Bereich 32. In Figuren 2 und 3 ist dies näher dargestellt.

Fig. 3 zeigt einen Schnitt entlang der Linie III-III in Fig. 1, d.h. einen Schnitt durch den Laryngoskopspatel 12 in einem Bereich distal von dem proximalen Abschnitt 32. In Umfangsrichtung erstreckt sich der Laryngoskopspatel 12 bezüglich einer vertikalen Achse 34 (0°-Achse) im Uhrzeigersinn bis zu einer Achse 36 über einen Winkel von etwa 45° im Uhrzeigersinn. Die Längsöffnung 26 erstreckt sich von der Achse 36 bis zu einer weiteren Achse 38 (170°-Achse), weist demnach einen Öffnungswinkel 40 von etwa 125° auf.

Am proximalen Ende 14 hingegen erstreckt sich die Längsöffnung 26 von der 45°-Achse bis zu einer Achse 42 (205°-Achse), so daß der Öffnungswinkel 40 der Längsöffnung 26 am proximalen Ende 14 etwa 160° beträgt.

Vom Handgriff 18 ausgehend ist ein Lichtleiter 44 auf der geschlossenen rechten Seite des Laryngoskopspatels 12 außen vom proximalen Ende 14 bis zum distalen Ende 16 geführt. Am distalen Ende 16 tritt der Lichtleiter 44 in das Lumen des Laryngoskopspatels 12 ein, so daß ein lichtaustrittsseitiges Ende 46 des Lichtleiters 44 auf der Innenseite, d.h. im Lumen des Laryngoskopspatels 12, angeordnet ist. Der Lichtleiter 44 weist ein optisches Faserbündel auf, das in einem Röhrchen geführt ist.

Wenn ein Flachleiter verwendet wird, kann der Lichtleiter 44 auch vom proximalen Ende 14 bis zum distalen Ende 16 auf der Innenseite des Laryngoskopspatels 12 geführt sein, weil in diesem Fall der Lichtleiter 44 nicht zu einer Behinderung beim Durchschieben des Tubus durch das Lumen, d.h. den Innenraum des Laryngoskopspatels 12 führt.

Am distalen Ende 16 verjüngt sich der Laryngoskopspatel 12 zu einer flachen Spitze 48. Wie aus Fig. 5 hervorgeht, erstreckt sich die Spitze 48 über etwa die gesamte Breite des Laryngoskopspatels 12. Ein distales Ende der Spitze 48 weist eine wulstartige Verdickung 50 auf. Die Verdickung 50 erstreckt sich dabei etwa orthogonal zur Längsrichtung des Laryngoskopspatels 12.

Am distalen Ende 16 geht der Laryngoskopspatel 12 demnach von der runden Querschnittsform in eine flache, ebene Form über, wobei die Spitze 48 bei dem in Figuren 1 bis 5 gezeigten Ausführungsbeispiel eine im wesentlichen geradlinige Verlängerung der Oberseite 30 des Laryngoskopspatels 12 bildet.

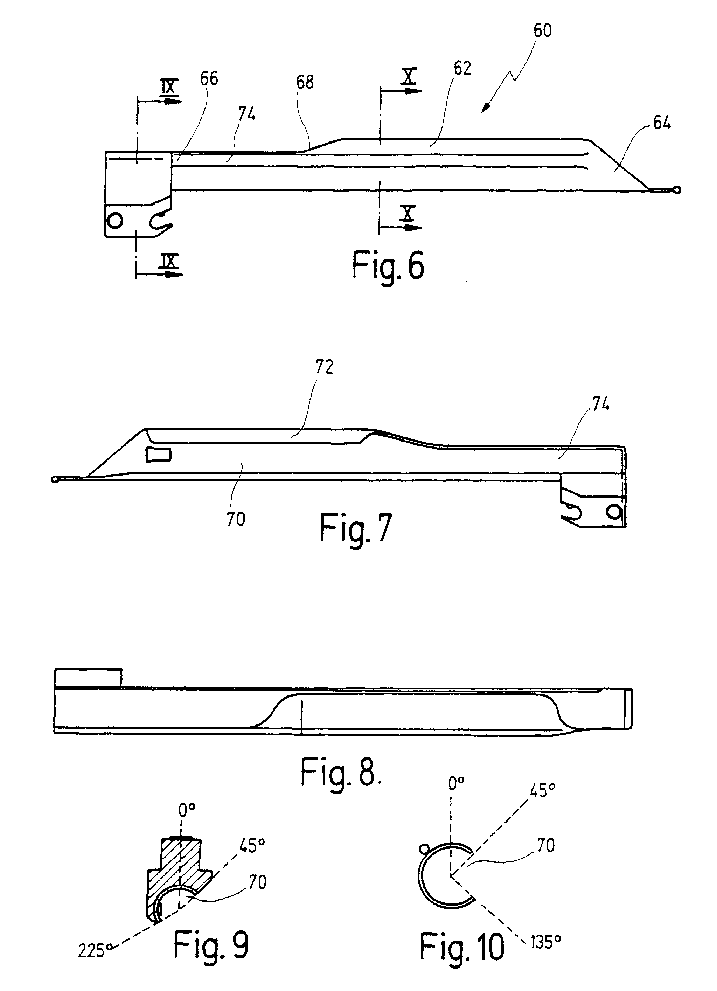

In Figuren 6 bis 10 ist ein weiteres Ausführungsbeispiel eines Laryngoskops 60 dargestellt. Das Laryngoskop 60 weist wiederum einen Laryngoskopspatel 62 auf, der wie bei dem Ausführungsbeispiel in Figuren 1 bis 5 im wesentlichen gerade ist.

Der Laryngoskopspatel 62 ist als rohrförmiger Hohlkörper ausgebildet, der im Querschnitt wiederum rund ist. Von einem distalen Ende 64 verjüngt sich der Laryngoskopspatel 62 im Querschnitt zu einem proximalen Ende 66 hin, wobei im Unterschied zu dem zuvor beschriebenen Ausführungsbeispiel gemäß Figuren 1 bis 5 die Querschnittsverjüngung, die in einem Übergangsbereich 68 zwischen dem distalen Ende 64 und dem proximalen Ende 66 stärker ausgeprägt ist als bei dem zuvor beschriebenen Ausführungsbeispiel. Die Querschnittsverjüngung in dem Übergangsbereich 68 ist sanft, so daß der Laryngoskopspatel 62 frei von Kanten ist, die traumatisch wirken können. Im Unterschied zu dem zuvor beschriebenen Ausführungsbeispiel ist außerdem der im Querschnitt verjüngte proximale Bereich des Laryngoskopspatels 62 länger ausgebildet.

Ein weiterer Unterschied zu dem Ausführungsbeispiel gemäß Figuren 1 bis 5 betrifft eine Längsöffnung 70, die in dem Laryngoskopspatel 62 zwischen dem distalen Ende 64 und dem proximalen Ende 66 ausgebildet ist. Im Unterschied zu dem zuvor beschriebenen Ausführungsbeispiel gemäß Figuren 1 bis 5 reicht die Längsöffnung 70 im distalen Bereich 72 in Umfangsrichtung von der 45°-Achse bis zu einer 135°-Achse, weist demnach einen Öffnungswinkel von etwa 90° auf. Bei diesem Ausführungsbeispiel ist die Längsöffnung 70 im Vergleich zu der Längsöffnung 26 des Laryngoskopspatels 12 in Umfangsrichtung somit schmaler. In einem proximalen Bereich 74 weitet sich die Öffnung 70 in Umfangsrichtung gegenüber dem distalen Bereich 72 bis zu einer 225°-Achse (vgl. Fig. 9) auf. Der Öffnungswinkel der Längsöffnung 70 im proximalen Bereich beträgt daher etwa 180° und ist größer als bei dem entsprechenden Abschnitt des Larynkoskopspatels 12.

Das Laryngoskop 10 gemäß Figuren 1 bis 5 und das Laryngoskop 60 gemäß Figuren 6 bis 10 dient jeweils dazu, einen Tubus durch das Lumen des Laryngoskopspatels 12 bzw. 62 in die Luftröhre eines Patienten einzuführen. Das Einführen des Tubus in das Lumen der Laryngoskopspatel 12 bzw. 62 kann dabei standardmäßig vom proximalen Ende aus durch das Lumen der Laryngoskopspatel 12 bzw. 62 erfolgen, oder über eine Einführhilfe (Boogie), und zwar jeweils unter Sichtkontrolle durch den Kehlkopf in die Luftröhre.

Zusätzlich zu diesen beiden Standardverfahren, bei denen das Laryngoskop auf der rechten Seite des Mundes des Patienten eingeführt wird, erleichtert es der Laryngoskopspatel 62, dessen Längsöffnung 70 im Vergleich zu dem Ausführungsbeispiel gemäß Figuren 1 bis 5 schmaler ausgebildet ist, die beiden zuvor erwähnten Verfahren auch auf der linken Seite der Zunge des Patienten durchzuführen.

Der Laryngoskopspatel 12 gemäß Figuren 1 bis 5, dessen Längsöffnung 26 weiter ausgebildet ist, ermöglicht zusätzlich zu den beiden Standardverfahren eine Durchfuhr eines Tubus unter Sichtkontrolle seitlich von der offenen Seite des Lumens des Laryngoskopspatels 12 her.

Je nach Patient kann es angezeigt sein, das Laryngoskop 10 gemäß Figuren 1 bis 5 oder das Laryngoskop 60 gemäß Figuren 6 bis 10 zu verwenden.

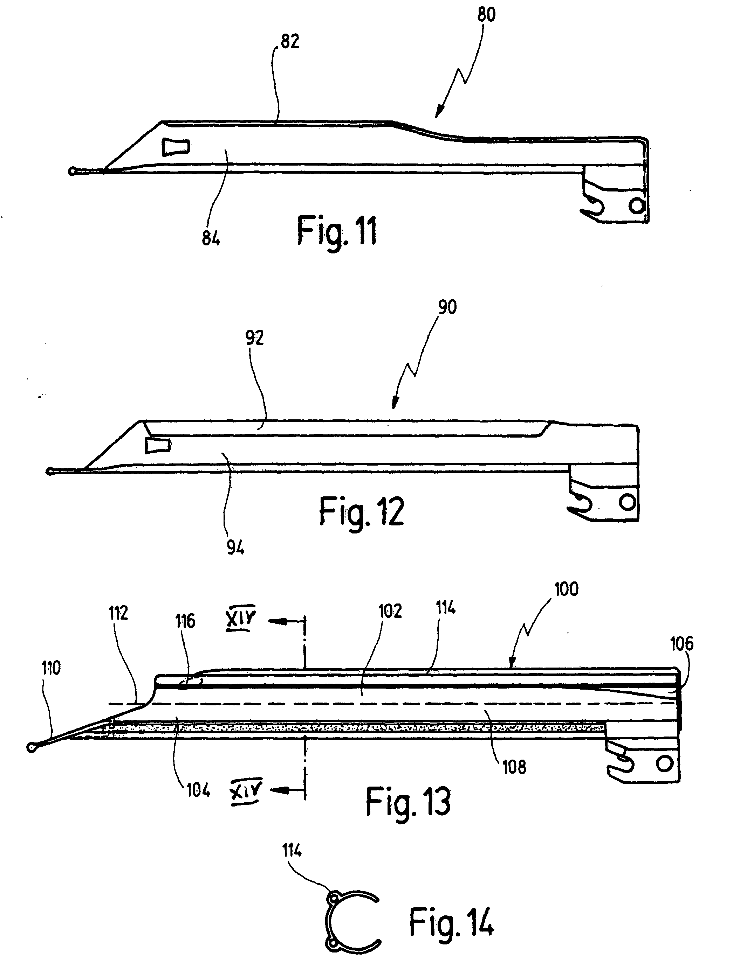

In Fig. 11 ist ein weiteres Ausführungsbeispiel eines Laryngoskops 80 dargestellt. Das Laryngoskop 80 weist einen Laryngoskopspatel 82 auf, der hinsichtlich seiner Geometrie eine Kombination der Laryngoskopspatel 12 und 62 darstellt. Mit dem Laryngoskopspatel 62 hat der Laryngoskopspatel 82 gemeinsam, daß sich der Querschnitt zum proximalen Ende hin stärker verjüngt, und daß der proximale Bereich kleineren Querschnitts lang ausgebildet ist. Mit dem Laryngoskopspatel 12 hat der Laryngoskopspatel 82 gemeinsam, daß eine Längsöffnung 84 in dem Laryngoskopspatel 82 im distalen Bereich einen insgesamt weiten Öffnungswinkel besitzt.

In Fig. 12 ist noch ein weiteres Ausführungsbeispiel eines Laryngoskops 90 dargestellt, das einen Laryngoskopspatel 92 aufweist, in dem wiederum eine Längsöffnung 94 vorgesehen ist. Die Geometrie des Laryngoskopspatels 92 ist wiederum eine Kombination der Geometrie des Laryngoskopspatels 12 gemäß Figuren

1 bis 5 und des Laryngoskopspatels 62 gemäß Figuren 6 bis 10. Im Unterschied zu dem Ausführungsbeispiel gemäß Fig. 11 ist hier jedoch die Längsöffnung 64 im distalen Bereich wie bei dem Ausführungsbeispiel gemäß Figuren 6 bis 10 schmaler ausgebildet, während wie bei dem Ausführungsbeispiel gemäß Figuren 1 bis 5 die Querschnittsverjüngung des Laryngoskopspatels 92 zum proximalen Ende hin weniger stark ausgeprägt ist.

In Figuren 13 und 14 ist schließlich noch ein letztes Ausführungsbeispiel eines Laryngoskops 100 dargestellt. Das Laryngoskop 100 weist einen Laryngoskopspatel 102 auf, der wiederum eine sich von einem distalen Ende 104 bis zu einem proximalen Ende 106 erstreckende Längsöffnung 108 aufweist.

Im Unterschied zu den vorherigen Ausführungsbeispielen verjüngt sich der Laryngoskopspatel 102 am distalen Ende 104 zu einer flachen, flächigen Spitze 110, die schräg zu einer Längsachse 112 des Laryngoskopspatels 102 verläuft. Anstatt der in allen Ausführungsbeispielen dargestellten geraden Ausführung der Spitze kann die Spitze auch gekrümmt ausgebildet sein.

Weiterhin ist außen an dem Laryngoskopspatel 102 ein Insufflationskanal 114 angeordnet. Der Insufflationskanal 114 weist eine Mündung 116 im Bereich des distalen Endes 104 des Laryngoskopspatels 102 auf. Durch den Insufflationskanal 114 kann, falls eine Notbeatmung des Patienten erforderlich ist, Sauerstoff intermittierend und mit hohem Druck dem Patienten zugeführt werden. Ein solcher Insufflationskanal kann auch bevorzugt bei den zuvor beschriebenen Ausführungsbeispielen vorgesehen sein.

Bei allen gezeigten Ausführungsbeispielen sind die Laryngoskopspatel 12, 62, 82, 92 und 102 schmal bauend ausgebildet, um das Positionieren der Laryngoskopspatel in dem Mund- und Rachenraum eines Patienten zu erleichtern. Weiterhin ist das Lumen der jeweiligen Laryngoskopspatel an den Durchmesser des einzuführenden Tubus angepaßt. An den Laryngoskopspateln können bspw. am proximalen Ende Beschriftungen, bspw. in Millimetern Durchmesser angebracht sein, um dem Arzt die maximale Größe des für den jeweiligen Laryngoskopspatel geeigneten Tubus anzuzeigen.

## Patentansprüche

1. Laryngoskop, insbesondere zum Einführen eines Tubus in die Luftröhre, mit einem sich im wesentlichen gerade erstreckenden Laryngoskopspatel (12; 62; 82; 92; 102), und mit einem Handgriff (18), wobei der Laryngoskopspatel (12; 62; 82; 92; 102) zumindest teilweise als rohrförmiger Hohlkörper zum Durchführen eines Tubus ausgebildet ist, der eine seitliche, sich von einem proximalen Ende (14; 66; 106) zu einem distalen Ende (16; 64; 104) des rohrförmigen Hohlkörpers erstreckende Längsöffnung (26; 70; 84; 94; 108) aufweist, wobei sich an das distale Ende (16; 64; 104) des rohrförmigen Hohlkörpers eine Spitze (48; 110) anschließt, **dadurch gekennzeichnet, daß** die Spitze (48; 110) in Richtung quer zur Längsrichtung des Laryngoskopspatels (12; 62; 82; 92; 102) eben und flach ist und ihr distales Ende in Richtung quer zur Längsrichtung im wesentlichen gerade ausgeführt ist.

2. Laryngoskop nach Anspruch 1, **dadurch gekennzeichnet, daß** der Laryngoskopspatel (12; 62; 82; 92; 102) im Querschnitt etwa rund ist.

3. Laryngoskop nach Anspruch 1 oder 2, **dadurch gekennzeichnet, daß** sich der Laryngoskopspatel (12; 62; 82; 92; 102) in einem proximalen Bereich (32; 74) im Querschnitt verjüngt.

4. Laryngoskop nach einem der Ansprüche 1 bis 3, **dadurch gekennzeichnet, daß** sich die Längsöffnung (26; 70; 84; 94; 108) zum proximalen Ende (14; 66; 106) hin aufweitet.

5. Laryngoskop nach einem der Ansprüche 1 bis 4, **dadurch gekennzeichnet, daß** die Längsöffnung (26; 70; 84; 94; 108) im Querschnitt einen Öffnungswinkel (40) im Bereich von etwa 180° bis etwa 90° aufweist.

6. Laryngoskop nach einem der Ansprüche 1 bis 5, **dadurch gekennzeichnet, daß** die Spitze (48; 110) in Längsrichtung geringfügig gekrümmt oder gerade ist.

7. Laryngoskop nach einem der Ansprüche 1 bis 6, **dadurch gekennzeichnet, daß** sich die Spitze (48; 110) etwa über die gesamte Breite des Laryngoskopspatels (12; 62; 82; 92; 102) erstreckt.

8. Laryngoskop nach einem der Ansprüche 1 bis 7, **dadurch gekennzeichnet, daß** die Spitze (48; 110) eine wulstartige Verdickung (50) aufweist.

9. Laryngoskop nach einem der Ansprüche 1 bis 8, **dadurch gekennzeichnet, daß** entlang des Laryngoskopspatels (12; 62; 82; 92; 102) ein Lichtleiter (44) geführt ist, dessen distales Ende (46) im Lumen des rohrförmigen Hohlkörpers (12; 62; 82; 92; 102) mündet.

10. Laryngoskop nach einem der Ansprüche 1 bis 9, **dadurch gekennzeichnet, daß** das proximale Ende (14; 66; 106) des Laryngoskopspatels (12; 62; 82; 92; 102) einen Befestigungsabschnitt (20) zum Verrasten des Handgriffs (18) an dem Laryngoskopspatel (12; 62; 82; 92; 102) aufweist.

11. Laryngoskop nach einem der Ansprüche 1 bis 10, **dadurch gekennzeichnet, daß** am Laryngoskopspatel (12; 62; 82; 92; 102) ein Insufflationskanal (114) angeordnet ist.

## Claims

1. A laryngoscope, in particular for introducing a tube into the trachea, comprising a laryngoscope spatula (12; 62; 82; 92; 102) extending in substantially straight fashion and a handle (18), wherein the laryngoscope spatula (12; 62; 82; 92; 102) is configured, at least in part, as a tubular hollow body for leading through a tube, which has a lateral longitudinal opening (26; 70; 84; 94; 108) extending from a proximal end (14; 66; 106) to a distal end (16; 64; 104) of the tubular hollow body, wherein a tip (48; 110) adjoins the distal end (16; 64; 104) of the tubular hollow body, **characterized in that** the tip (48; 110) is plane and flat in the direction transverse to the longitudinal direction of the laryngoscope spatula (12; 62; 82; 92; 102) and a distal end of the tip is designed substantially straight in the direction transverse to the longitudinal direction.

2. The laryngoscope of Claim 1, **characterized in that** the laryngoscope spatula (12; 62; 82; 92; 102) is approximately circular in cross-section.

3. The laryngoscope of Claim 1 or 2, **characterized in that** the cross-section of the laryngoscope spatula (12; 62; 82; 92; 102) tapers in a proximal area (32; 74).

4. The laryngoscope of anyone of Claims 1 through 3, **characterized in that** the longitudinal opening (26; 70; 84; 94; 108) widens toward the proximal end (14; 66; 106).

5. The laryngoscope of anyone of Claims 1 through 4, **characterized in that** the longitudinal opening (26; 70; 84; 94; 108) has an opening angle (40), viewed in cross-section, in the range of approximately 180° to approximately 90°.

6. The laryngoscope of anyone of Claims 1 through 5, **characterized in that** the tip (48; 110) is slightly curved or straight in the longitudinal direction.

7. The laryngoscope of anyone of Claims 1 through 6, **characterized in that** the tip (48; 110) extends approximately over the whole width of the laryngoscope spatula (12; 62; 82; 92; 102).

8. The laryngoscope of anyone of Claims 1 through 7, **characterized in that** the tip (48; 110) exhibits a bead-like thicker portion (50).

9. The laryngoscope of anyone of Claims 1 through 8, **characterized in that** a light guide (44), whose distal end (46) opens into the lumen of the laryngoscope spatula (12; 62; 82; 92; 102), is guided along the laryngoscope spatula (12; 62; 82; 92; 102).

10. The laryngoscope of anyone of Claims 1 through 9, **characterized in that** the proximal end (14; 66; 106) of the laryngoscope spatula (12; 62; 82; 92; 102) comprises a mounting section (20) for snap-locking the handle (18) on the laryngoscope spatula (12; 62; 82; 92; 102).

11. The laryngoscope of anyone of Claims 1 through 10, **characterized in that** an insufflation channel (114) is arranged on the laryngoscope spatula (12; 62; 82; 92; 102).

## Revendications

1. Laryngoscope, en particulier pour l'introduction d'un tube dans la trachée artère, avec une spatule laryngoscopique (12 ; 62 ; 82 ; 92 ; 102) s'étendant sensiblement en ligne droite, et avec une poignée (18), la spatule laryngoscopique (12 ; 62 ; 82; 92; 102) étant conformée au moins partiellement en corps creux tubulaire pour faire passer un tube, qui présente une ouverture longitudinale (26 ; 70 ; 84 ; 94 ; 108) latérale s'étendant d'une extrémité proximale (14 ; 66 ; 106) à une extrémité distale (16 ; 64 ; 104) du corps creux tubulaire, l'extrémité distale (16 ; 64 ; 104) du corps creux tubulaire étant suivie d'une pointe (48 ; 110), **caractérisé en ce que** la pointe (48 ; 110) est plate dans la direction transversale à la direction longitudinale de la spatule laryngoscopique (12; 62 ; 82; 92 ; 102) et son extrémité distale est réalisée pour l'essentiel droite dans la direction transversale à la direction longitudinale.

2. Laryngoscope selon la revendication 1, **caractérisé en ce que** la spatule laryngoscopique (12; 62 ; 82 ; 92 ; 102) présente une section transversale approximativement ronde.

3. Laryngoscope selon la revendication 1 ou 2, **caractérisé en ce que** la spatule laryngoscopique (12 ; 62 ; 82 ; 92 ; 102) est de section transversale effilée dans une partie proximale (32 ; 74).

4. Laryngoscope selon l'une des revendications 1 à 3, **caractérisé en ce que** l'ouverture longitudinale (26 ; 70 ; 84 ; 94 ; 108) s'élargit en direction de l'extrémité proximale (16 ; 66 ; 106).

5. Laryngoscope selon l'une des revendications 1 à 4, **caractérisé en ce que** l'ouverture longitudinale (26; 70 ; 84 ; 94; 108) présente en coupe transversale un angle d'ouverture (40) compris entre environ 180° et environ 90°.

6. Laryngoscope selon l'une des revendications 1 à 5, **caractérisé en ce que** la pointe (48 ; 110) est légèrement courbée ou droite dans la direction longitudinale.

7. Laryngoscope selon l'une des revendications 1 à 6, **caractérisé en ce que** la pointe (48 ; 110) s'étend approximativement sur toute la largeur de la spatule laryngoscopique (12 ; 62 ; 82 ; 92 ; 102).

8. Laryngoscope selon l'une des revendications 1 à 7, **caractérisé en ce que** la pointe (48 ; 110) présente un épaississement (50) de type bourrelet.

9. Laryngoscope selon l'une des revendications 1 à 8, **caractérisé en ce que** le long de la spatule laryngoscopique (12 ; 62 ; 82 ; 92 ; 102) passe un conducteur optique (44) dont l'extrémité distale (46) débute dans le lumen du corps creux tubulaire (12 ; 62 ; 82 ; 92 ; 102).

10. Laryngoscope selon l'une des revendications 1 à 9, **caractérisé en ce que** l'extrémité proximale (14 ; 66 ; 106) de la spatule laryngoscopique (12 ; 62 ; 82 ; 92 ; 102) présente une section de fixation (20) pour enclencher la poignée (18) sur la spatule laryngoscopique (12 ; 62 ; 82 ; 92 ; 102).

11. Laryngoscope selon l'une des revendications 1 à 10, **caractérisé en ce qu'**un canal d'insufflation (114) est placé sur la spatule laryngoscopique (12 ; 62 ; 82 ; 92 ; 102).
